# EUROPÄISCHE PATENTANMELDUNG

(11) **EP 1 872 805 A2**
(43) Veröffentlichungstag der Anmeldung: **02.01.2008**
(21) Anmeldenummer: 07075493.2
(22) Anmeldetag: 19.06.2007
(51) Int. Cl.: A61L 27/30

(54) **Beschichtetes Implantat**

(30) Priorität: 27.06.2006 DE 202006010202 U; 29.11.2006 DE 202006018188 U
(71) Anmelder: Biomed Est., 9490 Vaduz (LI)
(72) Erfinder: Ihde, Stefan Dr., 8738 Uetliburg (CH)
(74) Vertreter: Radwer, Dieter

(57) **Zusammenfassung**

Die Erfindung betrifft ein enossales Implantat mit einer Beschichtung, die den Einheilungsprozess forciert und der Entmineralisierung des Knochens bei Herstellung der notwendigen Kavitäten für die Insertion des Implantates entgegenwirkt.

Ziel der Erfindung ist die Erhöhung des osmotischen Drucks in der unmittelbaren Umgebung eines frisch eingebrachte Implantates, um den bei Herstellung der Kavität entstandenen Verlust an Salzen und Mineralien der im Knochen und in der Knochenflüssigkeit enthaltenen chemischen Verbindungen und Mineralien zügig auszugleichen.

Erfindungsgemäß ist die Implantatoberfläche beispielsweise mit einer trockenen Kruste aus löslichen NaCl-Molekülen beschichtet, die sich nach der Insertion allmählich auflöst. Die hohe Salzkonzentration der Beschichtung führt intraossär zu einem erheblichen Zustrom an interstitieller, im Knochen vorhandener Flüssigkeit auf das Implantat, während die Salzionen der Beschichtung in den Knochen diffundieren und den Verlust an Mineralien ausgleichen. Hierbei wird das Implantat zugleich stabilisiert und eine frühe Besiedlung mit Bakterien durch die erhöhte Salzkonzentration verhindert.

## Beschreibung

Die Erfindung betrifft ein weiter verbessertes, den Einheilungsprozess forcierendes, beschichtetes enossales Implantat, das in eine in den Knochen eingebrachte Kavität eingesetzt wird.

Der Erhalt der Stabilität von enossalen Implantaten gegenüber dem Knochen, in den sie eingebracht wurden, stellt häufig ein klinisches Problem dar. So werden sowohl in der orthopädischen Chirurgie als auch in der zahnärztlichen und maxillo facialen Implantologie immer wieder Lockerungen von Implantaten beobachtet. Ein gewisser Teil dieser Lockerungen ist dabei auf Infektionen zurückzuführen. Ein Großteil der Lockerungen hat als Ursache jedoch die Überlastung des periimplantären Knochens. So lockern sich bei Knochenbruchplatten die am meisten belasteten Schrauben bzw. die Schrauben, die in den am wenigsten mineralisierten Bereichen positioniert sind, beispielsweise in Zugbereichen oder Flexionsbereichen des Knochens.

Die bisher bekannten Maßnahmen, mit denen diese unerwünschten Prozesse eingeschränkt oder verhindert werden sollen, bestehen darin, die Knochenneubildung im knöchernen Operationsgebiet zu fördern. So wurde unter anderem vorgeschlagen, durch Beschichtungen der Implantatoberfläche mit knochenfördernden Substanzen die Neubildung von Knochengewebe zu beschleunigen und zu stimulieren.

Derartige Verfahren und Vorschläge für die Beschichtung von Implantaten sind beispielsweise aus DE 600 19 752 T2, DE 196 30 034 A1 und DE 196 28 464 A1 bekannt. Die bisher bekannten Maßnahmen zur Beschichtung von Implantaten beziehen sich überwiegend auf die verbesserte Bereitstellung von Substraten für den Knochenaufbau, wie beispielsweise Tricalciumphosphat, Hydrohylapatit, Ca- und P-Verbindungen aller Art. Um die Bildung von neuem Knochengewebe zu beschleunigen und zu stimulieren, wurden ferner Maßnahmen zur verbesserten Versorgung des Knochens mit Blut getroffen und schließlich die vermehrte Bereitstellung von Wachstumshormonen und Peptiden aller Art, die den Knochenaufbau beschleunigen, vorgeschlagen.

Alle diese Bemühungen haben bisher nicht zu einem wirklich nutzbaren klinisch guten Ergebnis geführt und hatten in der Praxis keinen durchschlagenden Erfolg, da es mehrere Wochen bis Monate dauert, bis der neu gebildete Knochen auch wirklich mineralisiert und tragfähig geworden ist. Die erwähnten Lockerungen von Implantaten treten jedoch weitaus früher auf.

Für das Einbringen von enossalen Implantaten wird mit geeigneten Bohr- und Fräswerkzeugen ein sogenanntes Implantatbett im Kieferknochen hergestellt und bei den hierfür durchzuführenden Arbeiten mit einer Kühlflüssigkeit gearbeitet, die einerseits die entstehenden Bohr- und Frässpäne austrägt und anderseits zum Kühlen der eingesetzten Werkzeuge und des Knochens im Bereich der Kavität bzw. des Implantatbettes dient.

Nach dem Stand der Technik werden für die Kühlung physiologisch verträgliche, isotonische Kochsalzlösungen verwendet, wie sie im übrigen auch als Infusionslösungen bekannt sind und für Infusionen in Venen und Arterien entwickelt wurden. Diese Infusionslösungen sind ständig verfügbar und können kostengünstig bezogen werden. Der Begriff "physiologisch" verträgliche, "isotonische" Kochsalzlösung bedeutet, dass die betreffende Lösung im Hinblick auf das Blut eines Patienten physiologisch bzw. isotonisch ist und in der Regel eine Kochsalzkonzentration von 0,9 % aufweist, denn diese NaCl - Konzentration liegt im Blut vor. Eine entsprechende, physiologisch verträgliche Kochsalzlösung ist so in der Lage, das Blutvolumen in den Blutgefäßen eines Patienten in gewissen Grenzen relativ gut zu ersetzen.

Neben den physiologisch verträglichen, isotonischen Kochsalzlösungen werden ferner Glucose - Lösungen oder Ringer-Lactat als Kühlflüssigkeit eingesetzt.

Das gemeinsame Merkmal der vorgenannten Flüssigkeiten, die als Kühlflüssigkeit eingesetzt werden, besteht darin, dass die physiologische Konzentration von Ionen- bzw, Salzen in Bezug auf die Blutflüssigkeit eines Patienten gewählt wurde. Eine "physiologisch" verträgliche Lösung, mit einer NaCl-Konzentration von 0,9 % ist jedoch bei weitem nicht physiologisch für den Knochen. Die in der Knochenflüssigkeit vorliegende Konzentration an Ionen und Salzen von Natriumchlorid, Kalzium und Phosphor ist weitaus höher als die im Blut. Setzt man nun eine physiologisch verträgliche, isotonische Kochsalzlösung mit einer NaCl-Konzentration von 0,9 % als Kühlflüssigkeit bei den durchzuführenden Bohr- und Fräsarbeiten ein, kommt es letztlich zu einem Auslaugen resp. zu einer, für den Einhellungsprozess nachteiligen Entmineralisierung des Knochens, da die zum Ausgleich des Konzentrationsgradienten der Kühlflüssigkeit notwendigen Salze und anderweitige Verbindungen, die im Knochen in weitaus höherer Konzentration vorliegen, aus dem Knochen herausgelöst werden. Das Gebiet um die Kavität verliert auf diese Weise seine höhere Konzentration an Salzen und anderweitigen Verbindungen und der Knochen wird zusätzlich verstärkt geschwächt.

Es sind ferner Implantate bekannt, die vom Hersteller in eine, mit Kochsalzlösung gefüllte Primärverpackung verpackt sind und in dieser Form feucht in die Praxis oder in die Klinik geliefert werden. Bei der verwendeten Lösung handelt es sich wiederum um eine physiologische Kochsalzlösung mit einer NaCl-Konzentration von 0,9 %. Aufgrund dieser NaCl-Konzentration ist auch diese Ausführungsform nicht in der Lage, die vorgenannten Probleme zu lösen oder die Lösung dieser Probleme in vorteilhafter Weise zu beeinflussen. Einen Vorteil für die Knochenheilung bietet diese Verpackungsform und das unmittel vor dem Einsetzen aus der Verpackung entnommene und mit der Kochsalzlösung benetzte Implantat nicht.

Der Erfindung liegt die Aufgabe zugrunde, eine Oberflächenbeschichtung für Implantate anzugeben, deren Substanzen die regulären osmotischen Verhältnisse des Knochens im Bereich des inserierten Implantates schnellstmöglich wieder herstellen und den durch die Bohr- und Fräsprozesse ausgelaugten Knochen und durch die Einblutung reduzierten Partialdruck an Salzen im Knochengewebe ausgleichen.

Erfindungsgemäß wird die Aufgabe durch ein Implantat nach den Merkmalen von Anspruch 1 gelöst. Weitere vorteilhafte Merkmale der Erfindung ergeben sich aus den Unteransprüchen 2 bis 15.

Auf der Suche nach Substanzen, die in der Lage sind, im implantologischen Bereich die regulären osmotischen Verhältnisse des Knochens im Bereich des inserierten Implantates schnellstmöglich wieder herzustellen und den durch die Bohr- und Fräsprozesse ausgelaugten Knochen und durch die Einblutung reduzierten Partialdruck an Salzen im Knochengewebe auszugleichen, wurde gefunden, dass bereits eine dünne Beschichtung der Implantatoberfläche mit reinem Kochsalz eine solche, lokale Wirkung hervorruft. Gleiches gilt auch für eine lösliche Beschichtung mit CaP, CaS04 und anderen im Knochen mit hoher Konzentration vorhandenen Verbindungen. Diese Substanzen zeigen eine ähnliche Wirkung wie Kochsalz.

Entscheidend für die schnelle Wiederherstellung der regulären osmotischen Verhältnisse des Knochens im Bereich des inserierten Implantates ist eine massive lokale Erhöhung der Konzentration der auf der Implantatoberfläche aufgebrachten Substanzen und ihre rasche Löslichkeit. Die Substanzen der Beschichtung dürfen nicht fest auf der Oberfläche des Implantates haften bzw. aufgetragen sein, wie beispielsweise bei Hydroxylapatit-Beschichtungen, sondern müssen in der Lage sein, nach der Insertion leicht in das angrenzende osteonalen Systeme des Knochens hinein diffundieren zu können.

Es reicht auch nicht aus, wenn die Konzentration der Substanzen in der Beschichtung, beispielsweise der NaCl - Gehalt in der üblichen, physiologisch verträglichen Konzentration von 0,9 %, vorliegt, wie sie allgemein von Kochsalzlösungen her bekannt ist. Die Konzentration der Substanzen muss weit aus höher sein als ihre Konzentration im Knochen und in der Knochenflüssigkeit. So führt beispielsweise eine Beschichtung mit einer höheren NaCL-Konzentration dazu, dass intraossär ein erheblicher Zustrom an interstitieller, im Knochen vorhandener Flüssigkeit auf das Implantat zu erreicht wird, während die Salzionen der Beschichtung vom Implantat weg in den Knochen diffundieren.

Auf diese Weise gelingt es, unmittelbar nach der Implantation im Knochen wieder reguläre osmotische Verhältnisse in der direkten Umgebung des Implantats herzustellen. Gleichzeitig wird das Implantat stabilisiert. Eine erhöhte Salzkonzentration verhindert zudem die frühe Besiedlung mit an sich übiquitär vorkommenden Bakterien.

Eine bevorzugte Ausführungsform der Erfindung zeichnet sich dadurch aus, dass die Natriumchloridkonzentration in der Beschichtungslösung höher als 0,9 % ist. Inwieweit die vorgenannte Konzentration den Betrag von 0,9 % erfindungsgemäß übersteigt, richtet sich bevorzugt ausschließlich nach der im Knochen erwarteten bzw. vorgefundenen Konzentration an Salzen. Vorteilhaft wird die Natriumchloridkonzentration so gewählt, dass sie im wesentlichen mindestens der plasmalsotonischen bzw. isoosmotischen Konzentration in Bezug auf die entsprechende Knochenflüssigkeit entspricht. Bevorzugt wird daher eine Natriumchloridkonzentration in der Beschichtungslösung von mehr als 0,95 %, weiter bevorzugt von mehr als 1,0 % verwendet.

Bei einer weiteren bevorzugten Ausführungsform entspricht auch die Konzentration von Kaliumchlorid im wesentlichen mindestens der Konzentration von Kaliumchlorid in der Knochenflüssigkeit des Knochens, in den die Kavität eingebracht worden ist.

Nach einem weiteren Merkmal der Erfindung entspricht die Konzentration von Kalziumchlorid im wesentlichen mindestens der Konzentration von Kalziumchlorid des zu behandelnden Knochens bzw. der Knochenflüssigkeit im Bereich der Kavität.

Um eventuelle lokale Infektion ebenfalls bekämpfen oder ihr prophylaktisch vorbeugen zu können, kann es von Vorteil sein, die Substanzen in der Beschichtung mit Antibiotika zu kombinieren. Einzelne der erfindungsgemäß vorgeschlagenen Substanzen sind per se antibiotisch wirksam, z.B. Calciumsulfat, Calciumphosphat. Deswegen kann es ausreichen, auch mehrere der genannten Substanzen für die Beschichtung zu kombinieren.

Die erfindungsgemäße Beschichtung kann beispielsweise in der Weise hergestellt werden, indem man ein enossales Implantat, das möglichst über eine aufgeraute Oberfläche verfügen sollte, am Ende des Reinigungsvorganges in eine Kochsalzlösung in der gewünschten Konzentration eintaucht und anschließend vorsichtig trocknen lässt. Nach Abschluss der Trocknungsphase verbleibt eine dünne, krustenartige lösliche Schicht von reinem Kochsalz auf der Implantatoberfläche. Während des Setzens des Implantats und danach löst sich diese krustenartige Schicht in der Knochenflüssigkeit und im lokal vorliegenden Blut auf. Auf diese Weise entsteht ein Ort höherer Salzkonzentration in dem Knochen, der die regulären osmotischen Verhältnisse des Knochens im Bereich des inserierten Implantates wieder herstellt und den durch die Bohr- und Fräsprozesse ausgelaugten Knochen und durch die Einblutung reduzierten Partialdruck an Salzen im Knochengewebe ausgleicht.

Im Experiment konnte nachgewiesen werden, dass bereits eine auf der Implantatoberfläche aufgetrocknete, physiologische Kochsalzlösung grundsätzlich andere Wirkung auf die Umgebung des Implantats im Kieferknochen hat als ein Implantat, welches unmittelbar vor der Insertion einer feuchten resp. flüssigen, physiologischen Kochsalzlösung entnommen wurde. Die angetrockneten Salzmoleküle lösen sich erst nach der Insertion des Implantats allmählich auf, was in vorteilhafter Weise zu einer wesentlich höheren lokalen Salzkonzentration im Bereich des inserierten Implantats führt, die den Partialdruck erhöht und in kürzester Zeit wieder reguläre osmotische Verhältnisse herstellt.

Durch Messungen mittels Resonanzschwingungen konnte festgestellt werden, dass erfindungsgemäß beschichtete Implantate sich schneller im Knochen festigen ais unbeschichtete Implantate.

Der Vorteil der relativ einfachen erfindungsgemäßen Lösung besteht vor allem darin, dass die vorgeschlagenen Mittel nur auf die osmotischen Verhältnisse einwirken. Für die schnelle Herstellung regulärer osmotischer Verhältnisse im Bereich des inserierten Implantates unmittelbar nach dem Einsetzen sind keine Medikamente erforderlich. Die eingesetzten Wirkstoffe, Kochsalz/NaCl oder die Salze anderweitiger chemischer Verbindungen, die im Knochen oder in der Knochenflüssigkeit physiologisch vorhanden sind, sind körperidentisch und daher problemlos abbaubar. Es kann auch zu keiner echten Überdosierung kommen, da durch den Flüssigkeitszufluss zum beschichteten Implantat stets eine Reduzierung (Anpassung) der Salzkonzentration in der Beschichtung eintritt.

## Patentansprüche

1. Beschichtetes Implantat, **dadurch gekennzeichnet, dass** die Implantatoberfiäche mit einer trockenen, sich auflösenden Kruste aus NaCl-Molekülen oder aus Salzmolekülen anderweitiger chemischer Verbindungen, die im Knochen und/ oder der Knochenflüssigkeit physiologisch vorhanden sind, beschichtet ist.

2. Beschichtetes Implantat nach Anspruch 1, **dadurch gekennzeichnet, dass** die Beschichtung aus Salzen einer Kalciumverbindung besteht.

3. Beschichtetes Implantat nach Anspruch 1 und 2, **dadurch gekennzeichnet, dass** die Beschichtung aus Salzen einer Kalciumphosphat- oder Kalciumsulphat-Verbindung besteht.

4. Beschichtetes Implantat nach Anspruch 1, **dadurch gekennzeichnet, dass** die Beschichtung aus Salzen einer Kaliumverbindung besteht

5. Beschichtetes Implantat nach Anspruch 1, **dadurch gekennzeichnet, dass** die Salzkonzentration in der Beschichtung im wesentlichen mindestens dem Salzgehalt im behandelten Knochen oder der Knochenflüssigkeit entspricht.

6. Beschichtetes Implantat nach Anspruch 1, **dadurch gekennzeichnet, dass** die Salzkonzentration in der getrockneten Kruste der Beschichtung über der physiologischen Konzentration der betreffenden Salze im Knochen oder der Knochenflüssigkeit liegt.

7. Beschichtetes Implantat nach Anspruch 1, **dadurch gekennzeichnet, dass** die NaCl - Konzentration in der Beschichtungslösung größer als 0,9 % ist.

8. Beschichtetes Implantat nach Anspruch 1 und 7, **dadurch gekennzeichnet, dass** die NaCL - Konzentration in der Beschichtungslösung mehr als 0,95 % beträgt.

9. Beschichtetes Implantat nach Anspruch 1 und 7, **dadurch gekennzeichnet, dass** die NaCl - Konzentration größer als 1 % ist.

10. Beschichtetes Implantat nach Anspruch 1 und einem der Ansprüche 2 bis 9, **dadurch gekennzeichnet, dass** die Beschichtung im Prozess der Endreinigung des implantats erfolgt und zum Anschluss der Endreinigung das Implantat in eine entsprechend aufbereitete Salzlösung getaucht wird und die auf der Implantatoberfläche haftenden Anteile an Salzlösung abschließend zu trocknen sind.

11. Beschichtetes Implantat nach Anspruch 1 und 10, **dadurch gekennzeichnet, dass** die NaCl - Konzentration der Waschlösung vor dem Trocknen des Implantates zum Aufbringen der Beschichtung mindesten 0,9 % beträgt.

12. Beschichtetes Implantat nach Anspruch 1 und 10, **dadurch gekennzeichnet, dass** die NaCl - Konzentration der Waschlösung vor dem Trocknen des Implantates zum Aufbringen der Beschichtung 1,0 % bis 20 % beträgt.

13. Beschichtetes Implantat nach einem der Ansprüche 1 bis 12, **dadurch gekennzeichnet, dass** es in einer mit einer flüssigen NaCl - Lösung gefüllten Primärverpackung verpackt und die NaCl - Konzentration in der Lösung > 0,9 % ist.

14. Beschichtetes Implantat nach einem der Ansprüche 1 bis 13, **dadurch gekennzeichnet, dass** die NaCl - Lösung der Primärverpackung als Gel vorliegt.

15. Beschichtetes Implantat nach einem der Ansprüche 1 bis 14, **dadurch gekennzeichnet, dass** die wirksamen Substanzen der Beschichtung mit antibiotisch wirksamen Substanzen kombiniert sind.
